# EUROPEAN PATENT APPLICATION

(11) **EP 3 311 788 A1**
(43) Date of publication of application: **25.04.2018**
(21) Application number: 16811653.1
(22) Date of filing: 15.06.2016
(51) Int. Cl.: A61J 1/05, A61M 11/00

(54) **OCULAR INSTILLATION ASSISTING TOOL**

(30) Priority: 18.06.2015 JP 2015123264; 13.06.2016 JP 2016117385
(71) Applicant: Santen Pharmaceutical Co., Ltd., Higashiyodogawa-ku Osaka-shi Osaka 533-8651 (JP)
(72) Inventor: YAMADA, Hiroshi, Ikoma-shi Nara 630-0101 (JP)
(74) Representative: Müller-Boré & Partner Patentanwälte PartG mbB
(86) International application number: PCT/JP2016/067806
(87) International publication number: WO 2016/204179

(57) **Abstract**

An embodiment of the present invention provides an ocular instillation assisting device that allows for ocular instillation of an ophthalmic solution in a subject facing forward even in a case where a remaining amount of the ' ophthalmic solution has been decreased, as with a case where the remaining amount of the ophthalmic solution is large. The ocular instillation assisting device (100) in accordance with an embodiment of the present invention includes an assisting device body (20). The assisting device body (20) fixes, at one end thereof, an ophthalmic solution container (10) such that a nozzle portion (14) is at a lower position than a container body (11) in a state in which a cup (40) has determined a position to which the ophthalmic solution is to be administered. The assisting device body (20) rotatably supports, at the other end thereof, a lever (30) for causing discharge of the ophthalmic solution.

## Description

### Technical Field

The present invention relates to an ocular instillation assisting device.

### Background Art

As a method for administering an ophthalmic solution to an eye of a patient, it is general to drop the ophthalmic solution into an eye. When this method is employed, the patient needs to turn his/her face up. However, in a case where the patient has difficulty in turning his/her face up, an ophthalmic solution cannot be appropriately administered to the eye of the patient. In order to appropriately administer the ophthalmic solution to such a patient, the ophthalmic solution needs to be dropped into the eye of the patient while the patient is lying with his/her face up.

In light of the above, in order to make it possible to administer an ophthalmic solution to an eye of a patient who is facing forward, for example, Patent Literature 1 discloses an eye fluid applicator.

### Citation List

### [Patent Literature]

### [Patent Literature 1]

Published Japanese Translation of PCT International Application, *Tokuhyo,* No. 2002-502289 (Publication Date: January 22, 2002)

### Summary of Invention

### Technical Problem

However, in a case where an ophthalmic solution is administered to an eye of a patient who is facing forward, the ophthalmic solution in a container could not be completely used up depending on a shape of the container. For example, in the case of the eye fluid applicator disclosed in Patent Literature 1, an orifice of a nozzle portion of an ophthalmic solution container to be used and the center of a bottom surface of a container body are coaxially arranged along one axis (fluid delivery axis). Then, as a remaining amount of the ophthalmic solution decreases, a patient needs to accordingly turn his/her face upward more. This leads to a problem that a patient's demand cannot be met. Further, unlike in a case where a remaining amount of the ophthalmic solution is large, administration of the ophthalmic solution is not possible with respect to a patient who is facing forward in a case where the remaining amount of the ophthalmic solution has been decreased. This leads to a problem that the ophthalmic solution in the container body cannot be completely used up.

An embodiment of the present invention is attained in view of the above problems, and an object of an embodiment of the present invention is to provide an ocular instillation assisting device capable of satisfying the following demands (a) and (b) of a patient who cannot turn his/her face up (who cannot look upward): (a) an ophthalmic solution can be administered to a patient facing forward even in a case where a remaining amount of the ophthalmic solution has been decreased, as with a case where the remaining amount of the ophthalmic solution is large; and (b) the ophthalmic solution in a container body can be completely used up. Note that application of the ocular instillation assisting device in accordance with an embodiment of the present invention is not limited only to application to administration of an ophthalmic solution to humans but the ocular instillation assisting device is also applicable to administration of an ophthalmic solution to animals suffering from eye diseases.

### Solution to Problem

In order to solve the above problems, an ocular instillation assisting device in accordance with an embodiment of the present invention is an ocular instillation assisting device for administering an ophthalmic solution to a subject of administration who is not looking upward, the ocular instillation assisting device including: a lever; an ophthalmic solution container used for administration of the ophthalmic solution, the ophthalmic solution container including: a container body which contains the ophthalmic solution; and a press-down portion for causing the ophthalmic solution contained in the container body to be discharged through a discharge hole in a case where the subject of administration depresses the lever; an assisting device body supporting the ophthalmic solution container and also rotatably supporting the lever; and a positioning section, the lever depressing the press-down portion when the lever is rotated to the assisting device body, and the assisting device body supporting the ophthalmic solution container such that the discharge hole is at a position lower than the container body in a state in which the position of the discharge hole relative to an eye of the subject of administration is determined by the positioning section.

In the above arrangement, the ophthalmic solution container is fixed to the assisting device body such that a discharge position of the ophthalmic solution is at a lower position than the container body in a state in which the positioning member has determined a position to which the ophthalmic solution is to be administered. Accordingly, even in a case where a direction into which a patient turns his/her face changes to some extent, the ophthalmic solution contained in the container body can be completely used up.

Accordingly, it is possible to continuously administer the ophthalmic solution to a patient who is not turning his/her face up by use of a general container, even without use of a container having a delamination structure that is a type of container structure for allowing the ophthalmic solution contained in the container body to be completely used up.

What is more, the lever is rotatably supported by the assisting device body. This lever is intended to allow the ophthalmic solution to be discharged by depression of an ophthalmic solution discharging section. Accordingly, use of this lever makes it possible to cause the ophthalmic solution to be discharged from the ophthalmic solution container more easily with less force, as compared to a case where the ophthalmic solution discharging section is directly depressed without use of the lever.

The ocular instillation assisting device may be arranged such that the assisting device body supports the ophthalmic solution container at one end of the assisting device body, and rotatably supports the lever at the other end of the assisting device body.

This allows the ophthalmic solution to be discharged from the ophthalmic solution container with less force.

The ocular instillation assisting device may be arranged such that the assisting device body rotatably supports the lever at one end of the assisting device body and has a grip portion, which the subject of administration grips, at the other end of the assisting device body; and the assisting device body supports the ophthalmic solution container between the grip portion and the one end of the assisting device body.

In this configuration, the position at which the ophthalmic solution container is supported is close to the position of a fulcrum of the lever. Accordingly, force to be applied to the lever can be reduced. Consequently, it becomes possible to discharge the ophthalmic solution from the ophthalmic solution container with less force.

The ocular instillation assisting device is arranged preferably such that: the positioning section is made of a cup member including a cup; the cup member is provided with a through hole at a bottom surface of the cup member; and the cup member is attached to the assisting device body such that the through hole communicates with the discharge hole through which the ophthalmic solution in the ophthalmic solution container is discharged.

With the above arrangement, the position of the discharge hole of the press-down portion relative to an eye can be easily determined by simply putting the cup member around the eye.

The ocular instillation assisting device is arranged preferably such that the positioning section is a rim portion of an opening that is opened so as to expose the discharge hole, the opening being formed at one end of the assisting device body.

With the above arrangement, the position of the discharge hole of the press-down portion relative to an eye can be easily determined by simply putting the rim portion of the opening around the eye.

The ocular instillation assisting device is arranged preferably such that: the press-down portion is provided with the discharge hole; and a depression direction of the press-down portion is not parallel to a discharge direction in which the ophthalmic solution is discharged through the discharge hole.

The ocular instillation assisting device is arranged preferably such that an angle between the depression direction and the discharge direction is not more than 90°.

With the above arrangement, the ophthalmic solution can be reliably administered to an eye of a patient if the angle between the depression direction and the discharge direction is not more than 90°. In other words, in a case where the above angle is more than 90°, the patient needs to take a difficult posture. Therefore, the above angle is preferably not more than 90°.

### Advantageous Effects of Invention

An embodiment of the present invention advantageously makes it possible to carry out ocular instillation without a problem even in a case where a direction into which a patient (subject of administration) turns his/her face changes to some extent. Further, an embodiment of the present invention advantageously makes it possible to completely use up an ophthalmic solution contained in the container body, since the ophthalmic solution can be administered to a patient facing forward even in a case where a remaining amount of the ophthalmic solution has been decreased, as with a case where the remaining amount of the ophthalmic solution is large.

### Brief Description of Drawings

Fig. 1 is a view schematically illustrating an arrangement of an ocular instillation assisting device in accordance with Embodiment 1 of the present invention.
Fig. 2 is a view schematically illustrating an arrangement of an ophthalmic solution container for use in the ocular instillation assisting device illustrated in Fig. 1.
Fig. 3 is a perspective view schematically illustrating an assisting device body of the ocular instillation assisting device illustrated in Fig. 1.
(a) of Fig. 4 is a top view of the assisting device body illustrated in Fig. 3. (b) of Fig. 4 is a side view partially including a cross sectional view of the assisting device body illustrated in Fig. 3.
Fig. 5 is a perspective view schematically illustrating a lever of the ocular instillation assisting device illustrated in Fig. 1.
(a) of Fig. 6 is a top view of the lever illustrated in Fig. 5. (b) of Fig. 6 is a side view of the lever illustrated in Fig. 5.
Fig. 7 is a perspective view schematically illustrating a cup of the ocular instillation assisting device illustrated in Fig. 1.
(a) of Fig. 8 is a right side view of the cup illustrated in Fig. 7. (b) of Fig. 8 is an elevational view of the cup illustrated in Fig. 7. (c) of Fig. 8 is a left side view of the cup illustrated in Fig. 7. (d) of Fig. 8 is a top view of the cup illustrated in Fig. 7.
(a) of Fig. 9 is a top view illustrating a state in which the cup illustrated in Fig. 7 is attached to the assisting device body. and (b) of Fig. 9 is a side view partially including a cross sectional view of the state in which the cup illustrated in Fig. 7 is attached to the assisting device body.
(a) and (b) of Fig. 10 are views illustrating an operation of the ocular instillation assisting device.

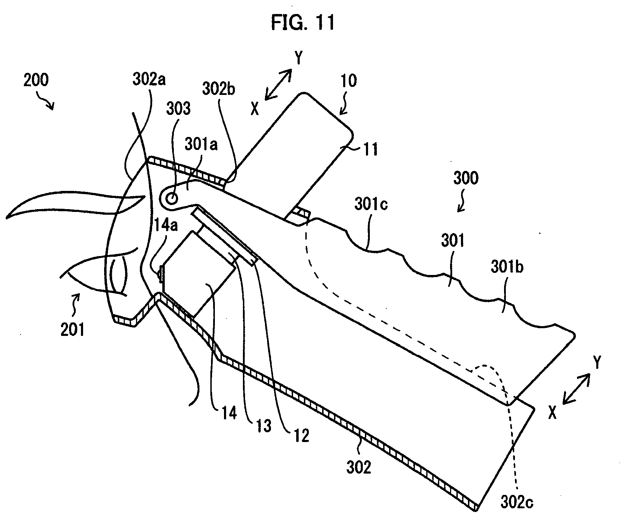

Fig. 11 is a view schematically illustrating an arrangement of an ocular instillation assisting device (another example) in accordance with Embodiment 2 of the present invention.

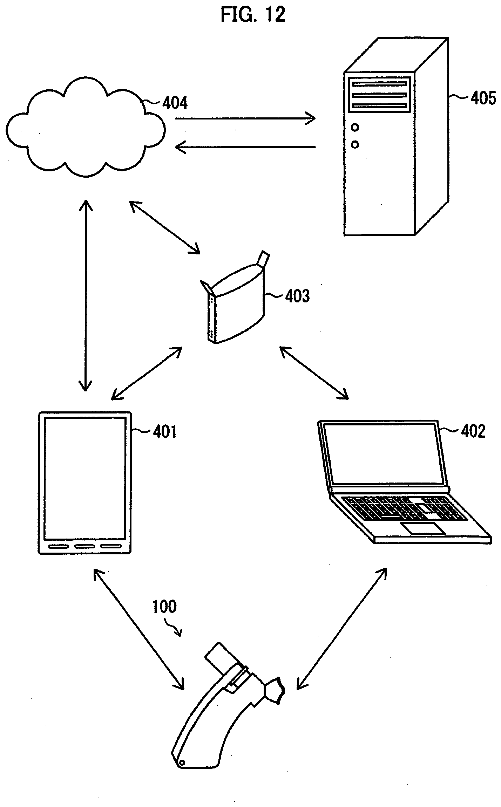

Fig. 12 is a block diagram schematically illustrating an arrangement of a medication management system in accordance with Embodiment 3 of the present invention.

### Description of Embodiments

### [Embodiment 1]

The following will discuss in detail an embodiment of the present invention.

First, the following will describe an ophthalmic solution container for use in an ocular instillation assisting device in accordance with an embodiment of the present invention.

### <Ophthalmic Solution Container>

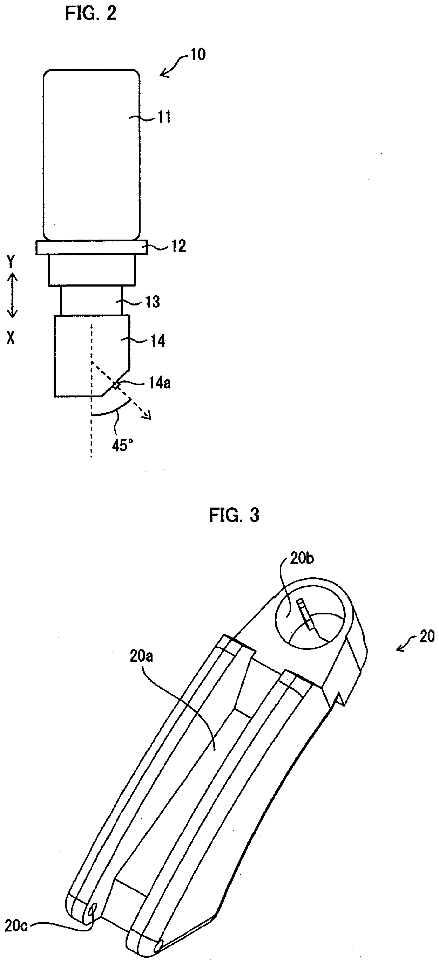

Fig. 2 is a view schematically illustrating an arrangement of an ophthalmic solution container (container) 10.

As illustrated in Fig. 2, the ophthalmic solution container 10 includes: a substantially cylindrical container body 11 for containing an ophthalmic solution; a flange 12 formed on the container body 11; a movable member 13 which is movable in an arrow X direction and an arrow Y direction with respect to the container body 11 and provided with an built-in mechanical pump; and a nozzle portion (press-down portion) 14 provided with a discharge hole 14a for discharging the ophthalmic solution that has been pumped from the container body 11 by the mechanical pump of the movable member 13.

By depressing the flange 12 in the arrow X direction, the movable member 13 moves in the arrow Y direction, so that the ophthalmic solution contained in the container body 11 is pumped. Then, the ophthalmic solution is discharged through the discharge hole 14a of the nozzle portion 14.

The discharge hole 14a is formed so as to allow the ophthalmic solution pumped by the movable member 13 to be discharged in a direction (discharge direction) tilted at 45° with respect to a movable direction (depression direction) of the movable member 13. Note that the discharge direction tilted at 45° as above is merely one example and the discharge direction is tilted preferably at not more than 90° with respect to the movable direction. Note also that such tilt of the discharge direction is set as appropriate in accordance with a shape etc. of an ocular instillation assisting device 100.

In the ophthalmic solution container 10 arranged as above, the ophthalmic solution in the container body 11 is pumped by the movable member 13 when the container body 11 is depressed toward the nozzle portion 14. Then, the ophthalmic solution is discharged to the outside through the discharge hole 14a of the nozzle portion 14. For example, in the case of the ophthalmic solution container 10, 5 µL to 30 pL of the ophthalmic solution can be discharged through the discharge hole 14a by moving the movable member 13. Note that a discharge amount of the ophthalmic solution can be adjusted by changing a size of the discharge hole 14a, a shape of an assisting device body 20, and/or the like.

Next, the following will describe an ocular instillation assisting device which uses the ophthalmic solution container 10 arranged as above.

### <Overview of Ocular Instillation Assisting Device>

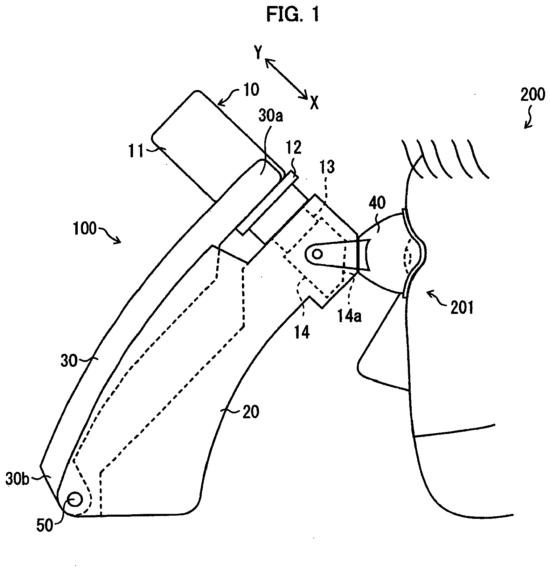

Fig. 1 is a view schematically illustrating an arrangement of the ocular instillation assisting device 100 in accordance with Embodiment 1.

The ocular instillation assisting device 100 is a device for assisting administration of an ophthalmic solution to a patient (subject of ocular instillation) 200. As illustrated in Fig. 1, the ocular instillation assisting device 100 includes the assisting device body (main body) 20, a lever 30, and a cup 40.

The assisting device body 20 fixes, at one end, the ophthalmic solution container 10 and at the other end, rotatably supports the lever 30 for causing the ophthalmic solution in the ophthalmic solution container 10 to be discharged. The assisting device body 20 will be described in detail later.

The lever 30 is arranged such that at one end portion 30a of the lever 30, the lever 30 holds the ophthalmic solution container 10 in cooperation with the assisting device body 20 and at the other end portion 30b of the lever 30, the lever 30 is rotatably supported with use of a shaft 50 by the assisting device body 20. When the lever 30 rotates to the assisting device body 20, the one end portion 30a comes, from above, into contact with the flange 12 which is provided on the container body 11 of the ophthalmic solution container 10 fixed to the assisting device body 20. The lever 30 will be described in detail later.

The cup 40 is attached to the assisting device body 20. This cup 40 is a cup member constituting a positioning section for determining a position of the discharge hole 14a of the nozzle portion 14 relative to an eye 201 of the patient 200. The cup 40 will be described in detail later.

### <Assisting Device Body>

Fig. 3 is a perspective view of the assisting device body 20.

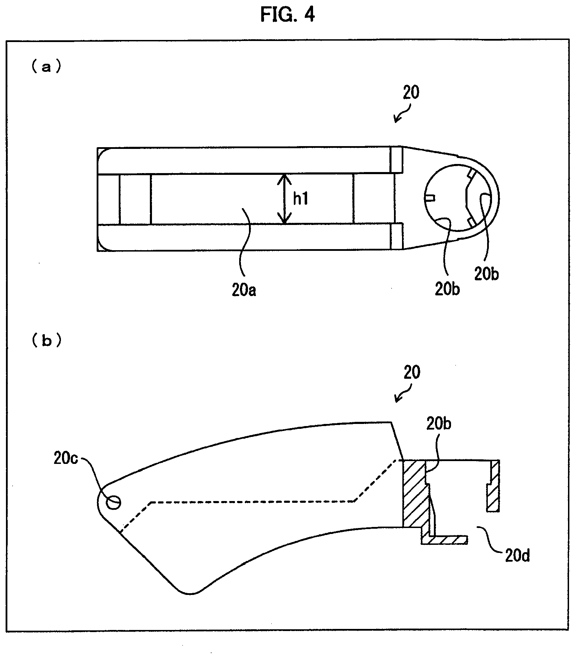

(a) of Fig. 4 is a top view of the assisting device body 20. (b) of Fig. 4 is a side view of the assisting device body 20.

The assisting device body 20 is made of resin. As illustrated in (a) of Fig. 4, in the assisting device body 20, a groove 20a is formed so as to have a predetermined width and so as to extend on a top surface of the assisting device body 20 in a longitudinal direction of the assisting device body 20. Further, a hollow portion 20b is formed on one end side of the groove 20a, and a support hole 20c is formed on the other end side of the groove 20a.

The groove 20a is formed so as to have a width h1, as illustrated in (a) of Fig. 4, such that the width h1 is the same as or a little larger than a width of the lever 30 described above. This allows the lever 30 to fit in the groove 20a when the lever 30 is rotated to the assisting device body 20.

The hollow portion 20b is arranged to fix the container body 11 when the nozzle portion 14 is inserted into the hollow portion 20b. As illustrated in Fig. 3 and (a) and (b) of Fig. 4, the hollow portion 20b is provided with an opening 20d for exposing the discharge hole 14a at the time when the nozzle portion 14 is fixed.

### <Lever>

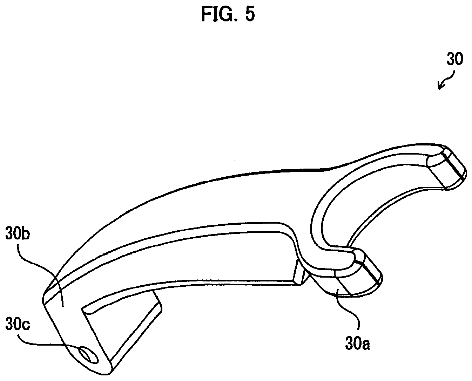

Fig. 5 is a perspective view of the lever 30.

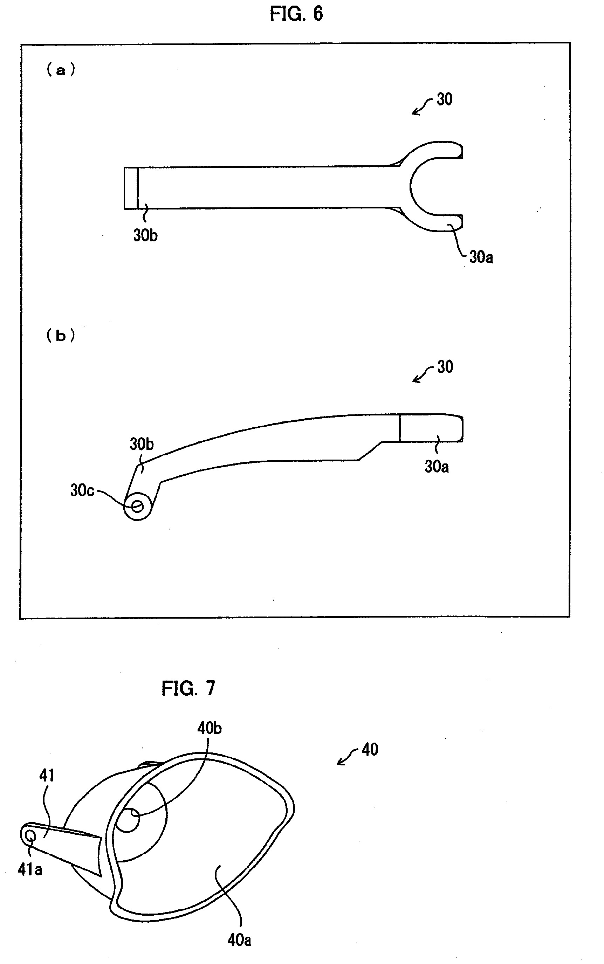

(a) of Fig. 6 is a top view of the lever 30; and (b) of Fig. 6 is a side view of the lever 30.

As illustrated in Fig. 5, the one end portion 30a of the lever 30 is formed so as to be bifurcated. With this shape, the ophthalmic solution container 10 is held at an outer peripheral surface by the lever 30. Note that as illustrated in (a) of Fig. 6, a holding width h2 of the one end portion 30a is a little larger than a diameter of the container body 11 of the ophthalmic solution container 10 but smaller than a diameter of the flange 12. This allows the one end portion 30a to stop at a top surface of the flange 12, when the lever 30 rotates and moves from above the container body 11.

At the other end portion 30b of the lever 30, a support hole 30c is formed. The support hole 30c is overlapped with the support hole 20c of the assisting device body 20, and then, the shaft 50 is provided through the support hole 30c and the support hole 20c. This allows the lever 30 to be rotatably supported by the assisting device body 20.

### <Cup>

Fig. 7 is a perspective view of the cup 40.

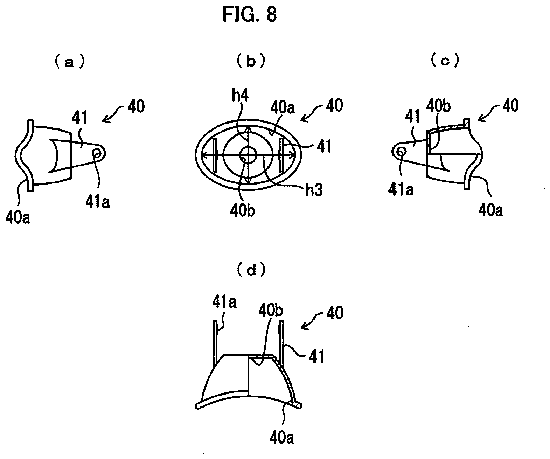

(a) to (d) of Fig. 8 are four orthogonal views of the cup 40.

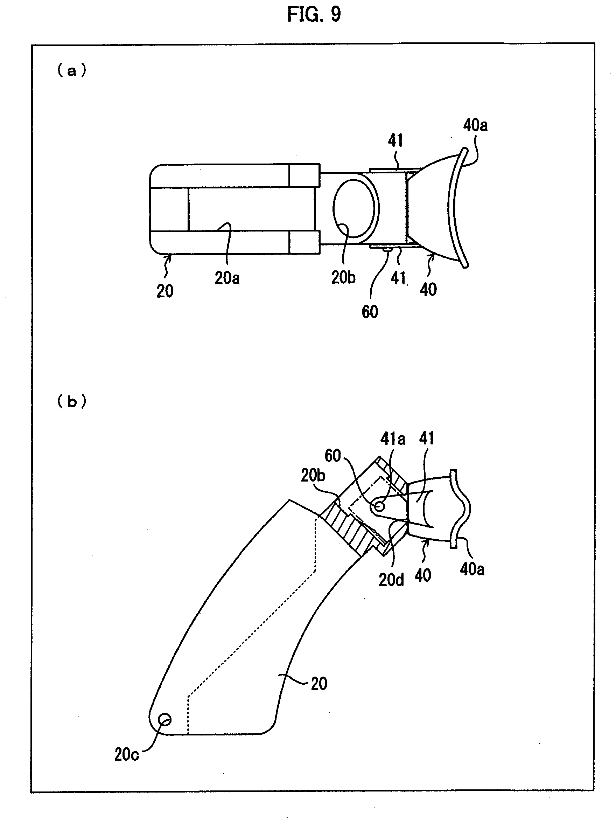

(a) and (b) of Fig. 9 are views illustrating a state in which the cup 40 is attached to the assisting device body 20.

The cup 40 is made of a polymeric material such as silicone. As illustrated in Fig. 7, the cup 40 is provided with (i) an opening 40a whose peripheral part comes in contact with a surrounding area of an eye 201 of a patient 200, and (ii) a through hole 40b (see Fig. 8) formed on an opposite side of the opening 40a. This cup 40 is a member for guiding the ophthalmic solution having been discharged through the through hole 40b into the eye 201 of the patient 200. A shape of the peripheral part of the opening 40a is arranged such that the peripheral part of the opening 40a can be tightly fitted to the surrounding area of the eye 201 of the patient 200.

The cup 40 is attached to the assisting device body 20 so that the through hole 40b will communicate with the discharge hole 14a of the nozzle portion 14 through which the ophthalmic solution in the ophthalmic solution container 10 is to be discharged. The discharge direction of the ophthalmic solution discharged through the through hole 40b of the cup 40 is a direction tilted at a preset angle with respect to a direction in which the flange 12 serving as an ophthalmic solution discharging section of the ophthalmic solution container 10 is depressed. For example, in Fig. 2, the preset angle is 45° in accordance with 45° that is an angle defining the discharge direction of the ophthalmic solution from the ophthalmic solution container 10. However, the preset angle may be any angle in a range of 20° to 70°, in consideration of demands of patients, and/or the like.

As illustrated in (a) to (d) of Fig. 8, the cup 40 is provided with an attachment member 41, on an opposite side of the opening 40a. The attachment member 41 is a member for fixing the cup 40 to the assisting device body 20. At one end of the attachment member 41, a support hole 41a is formed. This support hole 41a is used for attaching the cup 40 to the assisting device body 20. Specifically, as illustrated in (a) and (b) of Fig. 9, a projection 60 is formed on a side surface of the assisting device body 20 on a side where the hollow portion 20b is provided. This projection 60 is fit in the support hole 41a of the attachment member 41 of the cup 40. This allows the cup 40 to be supported by the assisting device body 20.

The attachment member 41 is made of an elastic material. Then, when the cup 40 is attached to the assisting device body 20, the attachment member 41 is spread outward and the projection 60 of the assisting device body 20 is fit in the support hole 41a. In other words, the cup 40 can be detachably attached to the assisting device body 20 by use of the attachment member 41.

As described above, attaching the cup 40 detachably to the assisting device body 20 produces the following advantageous effects.

The opening 40a of the cup 40 has an elliptical shape as illustrated in (b) of Fig. 8. This allows the peripheral part of the opening 40a to fit to the surrounding area of the eye 201 of the patient 200. Note that in order to fit the cup 40 to the surrounding area of the eye 201 of the patient 200, it is necessary to, in view of difference in patients 200 such as difference between adults, children, mem, women, etc., (i) prepare a plurality of kinds of cups 40 which vary in size and/or shape, that is, a plurality of kinds of cups 40 whose openings 40a vary in size and/or shape and (ii) switch such cups 40 as needed. For example, a plurality of kinds of cups 40 are prepared such that the cups 40 have different diameters h3 in a long-side direction of the opening 40a and different diameters h4 in a short-side direction of the opening 40a, and then the cups 40 thus prepared are switched depending on age and sex of the patient 200. This allows the cup 40 to fit to the surrounding area of the eye 201 of the patient 200 depending on age and sex of the patient 200.

Here, if a distance from the through hole 40b of the cup 40 to the eye 201 is shortened, more accurate ocular instillation becomes possible. However, in this case, the cup 40 may touch the eye 201 and hurt the eye 201 or a tip portion of the cup 40 may be contaminated due to a contact of the cup 40 with eyelashes. In contrast, in a case where the distance is arranged to be too long, the ophthalmic solution having flied along a parabola due to gravity may miss a target if a flying power of the ophthalmic solution is weak. The distance from the through hole 40b of the cup 40 to the eye 201 is set in consideration of the above matters.

Note that in Embodiment 1, the distance from the through hole 40b of the cup 40 to the eye 201 of the patient 200 is set in a range of 1 mm to 25 mm, and more preferably in a range of 3 mm to 20 mm, in a case where the cup 40 is fit to the surrounding area of the eye 201 of the patient 200, for example, as illustrated in Fig. 1. This is intended to appropriately administer the ophthalmic solution to the eye 201.

### <Description of Operation of Ocular Instillation Assisting Device>

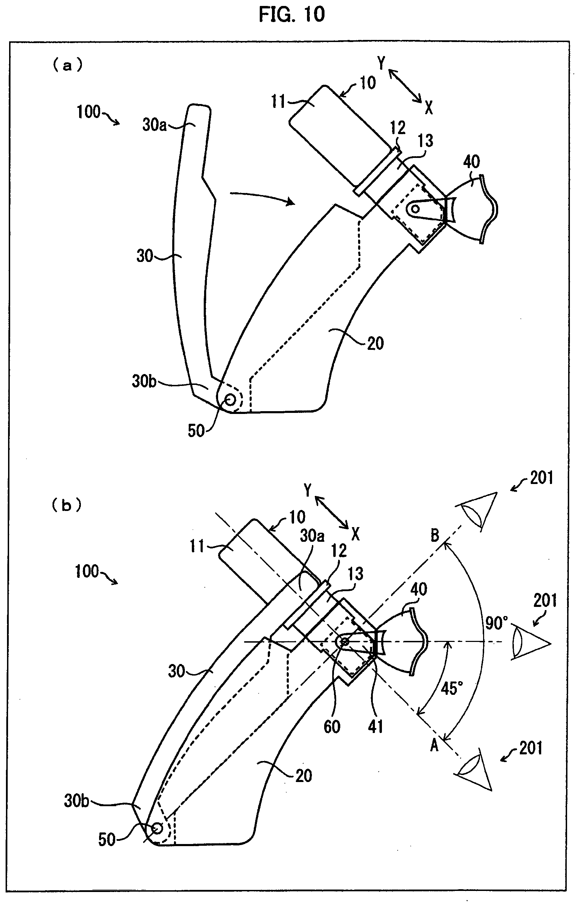

(a) and (b) of Fig. 10 are views illustrating an operation of the ocular instillation assisting device 100.

First, as illustrated in (a) of Fig. 10, the lever 30 attached to the assisting device body 20 is opened and the ophthalmic solution container 10 is fixed to a predetermined position of the assisting device body 20. In this case, the ophthalmic solution container 10 is inserted into the hollow portion 20b of the assisting device body 20 such that the nozzle portion 14 is located on a lower side. Then, the ophthalmic solution container 10 is fixed such that the discharge hole 14a of the nozzle portion 14 is positioned at the through hole 40b ((b) of Fig. 8) of the cup 40.

Next, as illustrated in (b) of Fig. 10, the lever 30 is rotated to the assisting device body 20, in other words, the lever 30 is rotated in the arrow X direction, so that the one end portion 30a of the lever 30 is brought into contact with the top surface of the flange 12 of the ophthalmic solution container 10. In this state, the lever 30 is further rotated in the arrow X direction so as to move the flange 12 in the arrow X direction. As a result, the movable member 13 is pressed into the container body 11, and the ophthalmic solution in the container body 11 is pumped with use of a pumping function. Consequently, the ophthalmic solution is discharged through the through hole 40b ((b) of Fig. 8) of the cup 40 from the discharge hole 14a of the nozzle portion 14. This allows for administration of the ophthalmic solution into the eye 201 of the patient 200 ahead of the cup 40.

In this manner, a subject of administration can administer an ophthalmic solution to himself/herself only by rotating the lever 30 to the assisting device body 20 while the subject of administration is facing forward. This allows, for example, a person who has a weak grip to easily administer an ophthalmic solution. Further, an ophthalmic solution can be administered to a subject who/which is not looking upward. Accordingly, an ophthalmic solution can be administered to a person who cannot turn his/her face up for administration of the ophthalmic solution.

Note that in Embodiment 1, the ophthalmic solution container 10 is provided with the discharge hole 14a of the nozzle portion 14 such that the depression direction of the movable member 13 makes an angle of 45° with the discharge direction of the ophthalmic solution, as illustrated in Fig. 2. In accordance with such an arrangement, the cup 40 is attached to the assisting device body 20, as illustrated in (b) of Fig. 10, so as to be positioned at an angle in the middle of a range between (i) a straight line passing through the shaft 50 and the shaft 60 and (ii) a line orthogonal to this straight line (a range of 90° between lines labeled "A" and "B", respectively). In other words, the cup 40 is attached to the assisting device body 20 so as to make an angle of 45° with each of the line labeled "A" and the line labeled "B". With this arrangement, the patient 200 can perform ocular instillation to the eye 201 while the patient 200 is facing forward. Note, however, the following points. The discharge direction of the ophthalmic solution from the cup 40 may be within a range of 90° between the lines labeled "A" and "B" as illustrated in, for example, (b) of Fig. 10. However, in a case where the discharge direction of the ophthalmic solution from the cup 40 becomes close to the line labeled "B", the patient needs to bend his/her neck downward. Then, in such a case, the patient needs to take a difficult posture when the patient performs ocular instillation to the eye 201. On the other hand, in a case where the discharge direction of the ophthalmic solution from the cup 40 becomes close to the line labeled "A", the patient needs to look upward when the patient performs optical instillation to the eye 201. In such cases, the primary object of an embodiment of the present invention (to allow for ocular instillation to an eye 201 of a subject who/which is not looking upward) cannot be achieved.

In addition, in a case where the container body 11 of the ophthalmic solution container 10 is arranged to have a delamination structure, the discharge direction of the ophthalmic solution from the ophthalmic solution container 10 does not need to be tilted at an angle of 45°, unlike the above arrangement illustrated in Fig. 2. Accordingly, it is not necessary to particularly consider an angle of a tilt of the discharge direction. This is for the following reason. In a case where the container body 11 of the ophthalmic solution container 10 is arranged to have a delamination structure, the ophthalmic solution can be completely used up even with an arrangement in which an orifice of a nozzle portion of the ophthalmic solution container and the center of a bottom surface of the container body are coaxially arranged along one axis (fluid delivery axis).

### <Effects>

In the ocular instillation assisting device 100 arranged as above, the ophthalmic solution container 10 is fixed to one end of the assisting device body 20 such that the nozzle portion 14 defining a discharge position of an ophthalmic solution is at a lower position than the container body 11 in a state in which the cup 40 as a positioning member has determined a position to which the ophthalmic solution is to be administered. Accordingly, even in a case where the ophthalmic solution is continuously administered to a subject who/which is facing forward, the ophthalmic solution contained in the container body 11 can be completely used up.

Accordingly, it is possible to continuously administer the ophthalmic solution to a subject who/which is not facing upward by use of a general container, even without use of a container having a delamination structure that is a type of container structure for allowing the ophthalmic solution contained in the container body to be completely used up.

What is more, the lever 30 is rotatably supported at the other end of the assisting device body 20. This lever 30 is intended to allow the ophthalmic solution to be discharged by depression of the flange 12 that is an ophthalmic solution discharging section. Accordingly, use of this lever 30 makes it possible to cause the ophthalmic solution to be discharged from the ophthalmic solution container more easily with less force as compared to a case where the flange 12 is directly depressed without use of the lever 30.

Though in Embodiment 1, the lever 30 is supported at a position on a lower side due to a shape of the assisting device body 20 as illustrated in Fig. 1, an embodiment of the present invention is not limited to this arrangement. The position (the other end of the assisting device body 20) at which the lever 30 is supported may be on an upper side as compared to the position at which the ophthalmic solution container 10 is fixed. In other words, the assisting device body 20 may be an ocular instillation assisting device which is arranged to have an upside-down shape of the shape illustrated in Fig. 1 such that the position at which the ophthalmic solution container 10 is fixed is on a lower side and the position at which the lever 30 is supported is on an upper side. This arrangement will be discussed in the following Embodiment 2.

### [Embodiment 2]

The following will discuss another embodiment of the present invention. Note that in Embodiment 2, the following will describe an example which uses the ophthalmic solution container 10 described in Embodiment 1.

### <Overview of Ocular Instillation Assisting Device>

Fig. 11 is a view schematically illustrating an arrangement of an ocular instillation assisting device 300 in accordance with Embodiment 2.

The ocular instillation assisting device 300 is a device for assisting administration of an ophthalmic solution to a patient (subject of ocular instillation) 200. As illustrated in Fig. 11, an ophthalmic solution container 10 for discharging the ophthalmic solution is mounted on the ocular instillation assisting device 300 and this ocular instillation assisting device 300 includes a lever 301 and an assisting device body (main body) 302.

An opening 302a is formed at one end portion of the assisting device body 302. A rim portion of the opening 302a functions as a cup for covering an eye 201 of the patient 200. That is, the opening 302a is a substitute of the cup 40 provided in the ocular instillation assisting device 100 illustrated in Fig. 1. In other words, the opening 302a as with the cup 40 is opened so as to expose a discharge hole 14a of a nozzle portion 14 of the ophthalmic solution container 10 so that the ophthalmic solution can be appropriately discharged to the eye 201 of the patient 200. The opening 302a functions as a positioning section for determining a position of the discharge hole 14a. Accordingly, the shape of the opening 302a is arranged such that a peripheral part of the opening 302a can be tightly fitted to a surrounding area of the eye 201 of the patient 200. Further, the opening 302a may be arranged such that the rim portion which comes in contact with the patient 200 is made of a polymeric material such as silicone. This is intended to improve tight fit with the patient 200 in a case where the rim portion comes in contact with the patient 200.

The assisting device body 302 rotatably supports the lever 301 on a side where the opening 302a is provided. Further, an opening 302b is formed at a position in the vicinity of the opening 302a. This opening 302b is an opening into which the ophthalmic solution container 10 is to be inserted. In addition, another opening 302c is formed on an opposite side of the opening 302b with respect to the opening 302a. The opening 302c is an opening into which a portion (the other end portion 301b) of the lever 301 is to be fit in.

The ophthalmic solution container 10 is loaded into the ocular instillation assisting device 300 such that in a state in which the peripheral part of the opening 302a of the ocular instillation assisting device 300 is tightly fitted to the surrounding area of the eye 201 of the patient 200, the discharge hole 14a of the nozzle portion 14 for discharging the ophthalmic solution is opposed to the eye 201 of the patient 200. Here, if a distance from the discharge hole 14a of the nozzle portion 14 to the eye 201 is shortened, more accurate ocular instillation becomes possible. However, in this case, the nozzle portion 14 may touch the eye 201 and hurt the eye 201 or a tip portion of the nozzle portion 14 may be contaminated due to a contact of the nozzle portion 14 with eyelashes. In contrast, in a case where the distance is arranged to be too long, the ophthalmic solution having flied along a parabola due to gravity may miss a target if a flying power of the ophthalmic solution is weak. The distance from the discharge hole 14a of the nozzle portion 14 to the eye 201 is set in consideration of the above matters.

Note that in Embodiment 2, the distance from the discharge hole 14a of the nozzle portion 14 to the eye 201 of the patient 200 is set in a range of 1 mm to 25 mm, and more preferably in a range of 3 mm to 20 mm, in a case where the peripheral part of the opening 302a of the assisting device body 302 is fit to the surrounding area of the eye 201 of the patient 200, for example, as illustrated in Fig. 11. This is intended to appropriately administer the ophthalmic solution to the eye 201.

At one end portion 301a of the lever 301, the lever 301 is rotatably supported by a support shaft 303 which is provided between the opening 302a and the opening 302b inside the assisting device body 302. Meanwhile, the other end 301b of the lever 301 is exposed from the opening 302c of the assisting device body 302 and is movable in an arrow-X direction and an arrow-Y direction.

The lever 301 holds, in cooperation the assisting device body 302, the ophthalmic solution container 10 at the one end portion 301a of the assisting device body 302. Then, the other one end portion 301b of the lever 301 is moved in the arrow-X direction and the arrow-Y direction. This causes the one end portion 301a of the lever 301 to come, from above, in contact with a flange 12 which is provided on a container body 11 of the ophthalmic solution container 10.

Further, recesses 301c are formed respectively at four positions at equal intervals on a surface of the other end portion 301b (on a surface on a side where a user grips) of the lever 301. The recesses 301c are preferably formed at positions that allow user's fingers to naturally fit in the recesses 301c, in a case where the user grips and holds the lever 301 and the assisting device body 302 of the ocular instillation assisting device 300. In other words, a grip portion which a user is to grip is formed by the recesses 301c of the lever 301 and a portion of the assisting device body 302 which portion is opposed to the opening 302c of the assisting device body 302. Note that the number, the shape and the positions of the recesses 301c are not limited to the above arrangement.

Therefore, the assisting device body 302 is arranged so as to rotatably support the lever 301 at one end of the assisting device body 302. Meanwhile, at the other end of the assisting device body 302, the assisting device body 302 is arranged to have the grip portion which the subject of administration is to grip. The assisting device body 302 at the other end is also arranged to support the ophthalmic solution container 10 between the grip portion and a position at which the lever 301 is supported.

Further, when the lever 301 and the assisting device body 302 are fit together, a shape of a cross section of the grip portion is preferably an oval shape. In particular, the shape of the cross section of the grip portion is preferably an oval shape having a long side in a direction in which the lever 301 and the assisting device body 302 are opposed to each other. This allows the user to easily grip the ocular instillation assisting device 300 when the user takes a body posture for ocular instillation.

### <Description of Operation of Ocular Instillation Assisting Device>

As illustrated in Fig. 11, the peripheral part of the opening 302a of the assisting device body 302 is tightly fitted to the surrounding area of the eye 201 of the patient 200 while the ophthalmic solution container 10 is loaded in the ocular instillation assisting device 300. In this state, the lever 300 is rotated to the assisting device body 302, in other words, the lever 300 is rotated in the arrow X direction, so that the one end portion 301a of the lever 301 is brought from above into contact with a top surface of the flange 12 of the ophthalmic solution container 10. Here, the one end portion 301a of the lever 301 serves as a fulcrum. In this state, the lever 301 is further rotated in the arrow X direction so as to move the flange 12 in the arrow X direction. As a result, the movable member 13 is pressed into the container body 11, and the ophthalmic solution in the container body 11 is pumped with use of a pumping function. Consequently, the ophthalmic solution is discharged through the discharge hole 14a of the nozzle portion 14. This allows for administering, to the eye 201 of the patient 200, the ophthalmic solution discharged through the discharge hole 14a of the nozzle portion 14.

In this manner, by use of the ocular instillation assisting device 300, as with use of the ocular instillation assisting device 100, a subject of administration can administer an ophthalmic solution to himself/herself only by rotating the lever 301 to the assisting device body 302 while the subject of administration is facing forward. This allows, for example, a person who has a weak grip to easily administer an ophthalmic solution. Further, an ophthalmic solution can be administered to a subject who/which is not looking upward. Accordingly, an ophthalmic solution can be administered to a person who cannot turn his/her face up for administration of the ophthalmic solution.

In addition, in the ocular instillation assisting device 300 arranged as above, a position of the fulcrum for rotation of the lever 301 is close to a position of the ophthalmic solution container 10 loaded in the assisting device body 302, that is, close to a position where the flange 12 of the ophthalmic solution container 10 is to be depressed. Accordingly, the lever 301 can be rotated to the assisting device body 302 with a smaller force as compared to a case using the ocular instillation assisting device 100 in which a position of a fulcrum for rotation is far from a position where a flange of an ophthalmic solution container 10 is to be depressed.

### [Embodiment 3]

The following will discuss still another embodiment of the present invention. Note that in Embodiment 3, the following will describe medication management by use of the ocular instillation assisting device 100 described in Embodiment 1 or by use of the ocular instillation assisting device 300 described in Embodiment 2.

### <Medication Management>

An actual medication state of each patient can be grasped by incorporating one or more of a camera, an LED (light emitting diode) light, and a sensor into an ocular instillation device such as the ocular instillation assisting device 100 or 300.

For example, in an arrangement in which an LED light and a camera are provided in the cup 40 of the ocular instillation assisting device 100 illustrated in Fig. 1, the LED light is turned on when a patient 200 grips the ocular instillation assisting device 100. Then, the patient 200 can look at the light turned on when the patient 200 performs ocular instillation. This can lead the patient 200 to successful ocular instillation without his/her eye 201 closed. Further, when an ophthalmic solution is discharged, an image of the eye 201 is taken. Accumulation of such images taken by the camera makes it possible to manage a state of ocular instillation of the patient. It is possible to provide an LED light and a camera in the opening 302a of the assisting device body 302 in the ocular instillation assisting device 300 illustrated in Fig. 11, as in the above cup 40.

Further, in a case where an infrared temperature sensor is used as a sensor to be mounted to the ocular instillation assisting device 100 or 300, it is possible to objectively detect whether or not ocular instillation has been successfully performed. Specifically, an infrared sensor such as the one mounted to Ocular Surface Thermographer (Tomey) is incorporated inside the cup 40 of or inside the opening 302a of the ocular instillation assisting device 100 or 300. Then, a change in temperature of the surface of an eye is measured for several seconds after ocular instillation. Since the temperature of the ophthalmic solution is generally room temperature whereas the temperature of the surface of an eye is approximately 37°C, a sudden temperature drop is detected at the surface of the eye at a moment of ocular instillation. This phenomenon is utilized to make it possible to detect, with use of an infrared temperature sensor, whether or not ocular instillation has been successfully performed.

Note that the temperature of the surface of an eye may be measured by use of a non-contact thermometer including a microsensor and an amplifier. In this case, since a response speed is 30 msec and therefore a high speed, a temperature change of the surface of an eye can be instantaneously detected when a liquid drop enters the eye. This makes it possible to more quickly detect whether or not ocular instillation has been successfully performed.

An actual medication state of a patient can be obtained by associating, with the date of ocular instillation, data obtained as a result of ocular instillation by use of the ocular instillation assisting device 100 or 300 and managing the data associated with the date of ocular instillation. Such data includes, for example, data indicative of an amount of ophthalmic solution instilled into an eye, data of images taken by a camera, data indicative of whether or not ocular instillation has been successfully performed, and/or the like.

For example, data as described above, which can be obtained as a result of ocular instillation, is not only stored in the ocular instillation assisting device 100 or 300 that is an ocular instillation device but also synchronized with a smartphone and a personal computer. Further, the data synchronized with the smartphone and the personal computer is automatically stored in a cloud server via telephone lines and/or the Internet. The following will discuss a specific medication management system.

### <Medication Management System>

Fig. 12 is a block diagram schematically illustrating a medication management system for management of ocular instillation to a patient by use of the ocular instillation assisting device 100 as an ocular instillation device. Note that though in this <Medication Management System>, the following will describe an example in which the ocular instillation assisting device 100 illustrated in Fig. 1 is used as the ocular instillation assisting device, the ocular instillation assisting device 300 illustrated in Fig. 11 may be alternatively used.

As illustrated in Fig. 12, the medication management system employing the ocular instillation assisting device 100 includes: a smartphone (mobile terminal) 401 that is to be operated by a person (parent) who performs medication management for a user (patient) who uses the ocular instillation assisting device 100; a personal computer 402 that is to be operated by another person (doctor) who performs medication management for the user (patient: subject of administration); a router 403 connected to the Internet; a cloud computer 404; and a management server 405.

### <Functions of Ocular Instillation Assisting Device 100>

The ocular instillation assisting device 100 is provided with a clock, a counter, a camera, a memory, an LED light, a battery, a battery indicator, a communication function (Bluetooth (registered trademark) 4.0, WiFi (registered trademark)), a bar-code reader function, and an alarm sounder function. These are provided to the ocular instillation assisting device 100 for medication management in a case where a specialized preparation is mounted in the ocular instillation assisting device 100. This makes it possible to perform basic medication management as below by use of the ocular instillation assisting device 100.
(1) Determination of kind of preparation: A bar code attached to an ophthalmic solution container 10 containing a specialized preparation is read by use of the camera and the bar-code reader function, and a kind of the specialized preparation is determined. Then, the kind of the preparation is automatically recorded when the ophthalmic solution container 10 is inserted into the ocular instillation assisting device 100.
(2) Indication of time for ocular instillation (alarm): At a preset time for medication, an alarm is sounded by use of the alarm sounder function of the ocular instillation assisting device 100. In this case, the alarm may be sounded by the smartphone 401 that manages the ocular instillation assisting device 100.
(3) Recording of time of ocular instillation: When ocular instillation is performed by use of the ocular instillation assisting device 100, (i) data of an image(s) taken by the camera and (ii) data obtained from the sensor and indicative of whether or not ocular instillation has been successfully performed are stored together with the date of ocular instillation.
(4) Auxiliary light at the time of ocular instillation: The LED light provided inside the cup 40 is turned on by putting a lever 30 of the ocular instillation assisting device 100 in a half-pressed state. Then, the patient looks at light of the LED light at the time of ocular instillation and consciously open his/her eye for that ocular instillation.
(5) Device ID management: Identification information is set for data management. The identification information is associated with the data obtained and is then stored in the memory.
(6) Ocular instillation calendar: Data arranged in a table (ocular instillation calendar) is created based on the data having been stored. In the table, the date of ocular instillation, the kind of preparation, the image data, a note, etc. are written as records of ocular instillation in chronological order.
(7) Medication profile data: Kinds and records of prescribed preparations (date of administration, dosage, etc.)
(8) Management of amount of residual drops: An amount of residual drops of the ophthalmic solution is managed by indication of the amount of residual drops in a container and a usable period.
(9) Management of remaining battery life for ocular instillation assisting device 100: A remaining battery life is indicated by a battery indicator. For example, in a case where a life of a battery is considered to be approximately one year, a warning is given one month before the end of the life of the battery.
(10) Data communication (Bluetooth or WiFi) is performed with the smartphone 401 and the personal computer 402 by use of the communication function.

### <Functions that Can Be Performed by Smartphone 401>

Use of functions of the smartphone 401 makes it possible to perform basic medication management as below.
(1) Perform data communication (Bluetooth or WiFi) with the ocular instillation device.
(2) Download cloud data (medication management data) from the cloud computer 404 and display the medication management data thus downloaded.
(3) Display the medication management data on a calendar.
(4) Display the kind of a preparation in use, the amount of residual drops of the preparation in use, the number of remaining days for which the preparation can be used, and the like.
(5) Sound an alarm at the time for medication of every preparation of a plurality of kinds of preparations.
(6) Automatically upload, to a cloud, image data etc. obtained by the ocular instillation assisting device 100.

### <Functions of Personal Computer 402>

Basically, the personal computer 402 can carry out the functions which the smartphone 401 can perform. The personal computer 402 can carry out at least the following functions in addition to the functions which the smartphone 401 can perform.
(1) Check information of a plurality of patients stored in the cloud computer 404.
(2) Separately grasp respective total prescription amounts of preparations of a patient who uses the ocular instillation assisting device 100.

### <Actual Example of Medication Management by Use of Medication Management System>

### (Operation of Ocular Instillation Assisting Device 100)

As a method for recognizing a preparation by the ocular instillation assisting device 100, there are preparation recognition by software, which is one method, and preparation recognition by hardware, which is another method. Note that no electrical power switch is provided to the ocular instillation assisting device 100 and a battery is mounted to the ocular instillation assisting device 100, and that the ocular instillation assisting device 100 is kept activated as long as an electrical power is supplied.

### (1) Method of Preparation Recognition by Software

The camera mounted to the ocular instillation assisting device 100 is directed to a bar code display section of a preparation. Then, the lever 30 is squeezed. Next, when the camera has read a target bar code, the ocular instillation assisting device 100 produces a sound "pipi". At this point of time, registration of the kind of the preparation is completed. The preparation having been registered is set in the ocular instillation assisting device 100 such that a nozzle of a container of the preparation is appropriately positioned.

### (2) Method of Preparation Recognition by Hardware

A projection is provided at the nozzle portion 14 of the ophthalmic solution container 10 containing the preparation. The position of the projection differs depending on kinds of preparations. This preparation is set in the ocular instillation assisting device 100 such that a nozzle of the nozzle portion 14 is appropriately positioned. This allows the ocular instillation assisting device 100 to recognize a kind of the preparation thus set, on the basis of the position of the projection provided at the nozzle portion 14. Then, the kind of the preparation is registered in the ocular instillation assisting device 100.

The user lightly grips the ocular instillation assisting device 100 in which the ophthalmic solution container 10 has been set. At this time, the ophthalmic solution container 10 contains the preparation whose kind is recognized by the above method (1) or (2). Then, the user brings the cup 40 of the ocular instillation assisting device 100 to the position of his/her eye. The cup 40 is provided with a protrusion (not illustrated) for forcibly opening an eyelid. This protrusion works so as to pull the eyelid downward when the user presses the cup to the position of the eye and lightly squeezes the lever. In this state, when the user further but still lightly squeezes the ocular instillation assisting device 100, the LED light provided at the bottom of the cup 40 is turned on. When the patient further squeezes the ocular instillation assisting device 100 strongly while the patient looks at light of the LED light, a drop of the ophthalmic solution is discharged from a tip of the nozzle of the nozzle portion 14.

Here, at the same time as a liquid drop is discharged in instantaneous response to a strong squeeze of the ocular instillation assisting device 100, a shutter of the camera provided at the bottom of the cup 40 clicks and thereby a photo is taken. At this time, the shutter clicks a plurality of times in a moment, so that a plurality of photos is taken. Alternatively, passage of the liquid drop is detected by the sensor provided in the cup 40 or a change in temperature of the surface of the eye is detected at the time when the liquid drop enters the eye. This makes it possible to monitor whether or not ocular instillation is successfully performed.

When ocular instillation has been completed, the ophthalmic solution container 10 is removed from the ocular instillation assisting device 100. Then, a bar code of a next preparation is read so that the next preparation can be mounted. Thereafter, ocular instillation is performed in the above-described manner. Subsequently, when ocular instillation of all preparations has been completed, an operation of ocular instillation is completed.

Subsequently, the ocular instillation assisting device 100 carries out Bluetooth synchronization or WiFi synchronization of data with the smartphone 401 which the parent or the patient himself/herself operates.

### (Operation of Smartphone 401)

It is assumed that a dedicated application for the ocular instillation assisting device 100 for use in the medication management system is installed in the smartphone 401.

By use of this application, communication between the smartphone 401 and the ocular instillation assisting device 100 becomes possible. When the communication is carried out, the application of the smartphone obtains an ID number assigned to the ocular instillation assisting device 100, so that the ocular instillation assisting device 100 is recognized.

Further, by use of the application, communication can be carried out, by using the ID number assigned to the ocular instillation assisting device 100 and a password, through telephone lines via the management server 405 and the cloud computer 404 in the medication management system.

In the application, a calendar function is set up. This makes it possible to register a prescribed preparation and to set the time for ocular instillation. In addition, the time for ocular instillation can be set in the ocular instillation assisting device 100 by causing synchronization with the ocular instillation assisting device 100.

Use of the application makes it possible to communicate with the ocular instillation assisting device 100 and to take in data of one or more of the time at which ocular instillation is carried out, the kind of preparation, an image(s) taken by the camera and/or the sensor, and the like. The data taken in the above-described manner can be stored both in the ocular instillation assisting device 100 and in the smartphone 401 for not less than approximately 3 months. Even in a case where communication failure occurs, data is stored individually in each of the ocular instillation assisting device 100 and the smartphone 401.

When the application is active, the smartphone 401 displays, on a calendar displayed on a display screen, a mark(s) indicative of whether or not ocular instillation of each preparation has been successfully performed. Further, the image(s) and record(s) taken by the camera and/or the sensor can be checked as needed.

### (Cloud Server)

The cloud server is constructed with use of the cloud computer 404 and the management server 405 on the Internet, and has a database function and an analysis display function. Data for each day or data for a predetermined period is loaded into the cloud server via the smartphone 401. Such loading of the data accumulated in the smartphone 401 is carried out by clicking "Store" displayed on the display screen of the smartphone.

The cloud server can be accessed from the smartphone 401 or the personal computer 402 by using the ID number of the ocular instillation assisting device 100 and the password.

In a case where the cloud server is accessed from the smartphone 401, an access can be made, by using a registered ID number, to only data of an individual associated with the ID number. When a doctor desires to browse data of a plurality of patients, a right to access is separately set so that individual information on each of the patients can be accessed. For example, a doctor can check such information on the personal computer 402 by causing a calendar and image data, which show an ocular instillation profile of each individual, to be displayed for browsing of cloud data. Further, a doctor can check, on the personal computer 402, the number of times of ocular instillation of each preparation and the amount of residual drops of each preparation as additional information. Further, when the amount of residual drops of the preparation becomes small, the doctor can find a display to move the doctor to prescribe.

Note that each of the ocular instillation assisting devices 100 and 300 can be applied not only to the patient 200 but also to animals such as dogs and cats suffering from eye troubles, as subjects of ocular instillation.

The present invention is not limited to the embodiments, but can be altered by a skilled person in the art within the scope of the claims. The present invention also encompasses, in its technical scope, any embodiment derived by combining technical means disclosed in differing embodiments.

### Industrial Applicability

Embodiments of the present invention are applicable to an ocular instillation assisting device for administering an ophthalmic solution to a subject who/which is not looking upward.

### Reference Signs List

- 10: ophthalmic solution container
- 11: container body
- 12: flange (press-down portion)
- 13: movable member (press-down portion)
- 14: nozzle portion (press-down portion)
- 14a: discharge hole
- 20: assisting device body
- 20a: groove
- 20b: hollow portion
- 20c: support hole
- 20d: opening
- 30: lever
- 30a: one end portion
- 30b: other end portion
- 30c: support hole
- 40: cup (positioning section)
- 40a: opening
- 40b: through hole
- 41: attachment member
- 41a: support hole
- 60: projection
- 100: ocular instillation assisting device
- 200: patient (subject of administration)
- 201: eye
- 300: ocular instillation assisting device
- 301: lever
- 301a: one end portion
- 301b: other end portion
- 301c: recess (grip portion)
- 302: assisting device body (grip portion)
- 302a: opening
- 302b: opening (positioning section)
- 302c: opening
- 303: support shaft
- 401: smartphone
- 402: personal computer
- 403: router
- 404: cloud computer (cloud server)
- 405: management server (cloud server)

## Claims

1. An ocular instillation assisting device for administering an ophthalmic solution to a subject of administration who is not looking upward,
the ocular instillation assisting device comprising:
a lever;
an ophthalmic solution container used for administration of the ophthalmic solution, the ophthalmic solution container including:
a container body which contains the ophthalmic solution; and
a press-down portion for causing the ophthalmic solution contained in the container body to be discharged through a discharge hole in a case where the subject of administration depresses the lever;
an assisting device body supporting the ophthalmic solution container and also rotatably supporting the lever; and
a positioning section,
the lever depressing the press-down portion when the lever is rotated to the assisting device body, and
the assisting device body supporting the ophthalmic solution container such that the discharge hole is at a position lower than the container body in a state in which the position of the discharge hole relative to an eye of the subject of administration is determined by the positioning section.

2. The ocular instillation assisting device as set forth in claim 1, wherein the assisting device body supports the ophthalmic solution container at one end of the assisting device body, and rotatably supports the lever at the other end of the assisting device body.

3. The ocular instillation assisting device as set forth in claim 1, wherein:
the assisting device body rotatably supports the lever at one end of the assisting device body and has a grip portion, which the subject of administration grips, at the other end of the assisting device body; and
the assisting device body supports the ophthalmic solution container between the grip portion and a position at which the lever is supported.

4. The ocular instillation assisting device as set forth in any one of claims 1 through 3, wherein:
the positioning section is made of a cup member including a cup;
the cup is provided with a through hole at a bottom surface of the cup; and
the cup is attached to the assisting device body such that the through hole communicates with the discharge hole.

5. The ocular instillation assisting device as set forth in any one of claims 1 through 3, wherein the positioning section is a rim portion of an opening that is opened so as to expose the discharge hole, the opening being formed at one end of the assisting device body.

6. The ocular instillation assisting device as set forth in any one of claims 1 through 5, wherein:
the press-down portion is provided with the discharge hole; and
a depression direction of the press-down portion is not parallel to a discharge direction in which the ophthalmic solution is discharged through the discharge hole.

7. The ocular instillation assisting device as set forth in claim 6, wherein an angle between the depression direction and the discharge direction is not more than 90°.
